(19) Europäisches Patentamt
European Patent Office
Office européen des brevets

(11) Numéro de publication: **0 040 560**
**B1**

# (12) FASCICULE DE BREVET EUROPEEN

(45) Date de publication du fascicule du brevet: **08.06.83**

(51) Int. Cl.³: **C 07 C 143/70**

(21) Numéro de dépôt: **81400725.8**

(22) Date de dépôt: **07.05.81**

## (54) Fabrication de chlorures d'alcane sulfonyles.

(30) Priorité: **16.05.80 FR 8010993**

(43) Date de publication de la demande: **25.11.81 Bulletin 81/47**

(45) Mention de la délivrance du brevet: **08.06.83 Bulletin 83/23**

(84) Etats contractants désignés: **BE CH DE GB IT LI LU NL**

(56) Documents cités:
**US-A-2 277 325**
**US-A-2 465 952**
**US-A-3 248 423**

(73) Titulaire: **SOCIETE NATIONALE ELF AQUITAINE (PRODUCTION), Tour Aquitaine, F-92400 Courbevoie (FR)**

(72) Inventeur: **Gongora, Henri, 12 rue Diraty, F-64140 Billere (FR)**
Inventeur: **Tournier-Lasserve, Jacques, 44 rue Castetnau, F-64000 Pau (FR)**

(74) Mandataire: **Kohn, Armand, 5 Avenue Foch, F-92380 Garches (FR)**

**EP 0 040 560 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Fabrication de chlorures d'alcane sulfonyles

La présente invention concerne un nouveau procédé pour la fabrication de chlorures d'alcane sulfonyles, liquides à la température ordinaire, et des acides correspondants.

Les chlorures d'alcane sulfonyles, en particulier le chlorure de méthane sulfonyle $ClCH_3SO_2$, sont des produits industriels intéressants: ils ont différentes applications, notamment comme intermédiaires dans la production de corticostéroïdes, de produits photographiques, pharmaceutiques, de colorants et d'autres composés chimiques. Aussi s'est-on depuis longtemps occupé de leur préparation. Dès 1938, DOUGLASS et JOHNSON (J. Amer. Chem. Soc. 1938, n°60, p. 1486–89) ont décrit la réaction de disulfures organiques et de mercaptans avec le chlore dans de l'acide chlorhydrique aqueux, donnant les chlorures des sulfonyles aryliques et aliphatiques; ils ont ainsi préparé les chlorures de benzène sulfonyle, d'éthane-sulfonyle, de n-pentane sulfonyle et de n-butane sulfonyle. Cette réaction, depuis lors, est appliquée industriellement, surtout à la fabrication du chlorure de méthane sulfonyle; le procédé généralement adopté comprend la mise en contact, dans le bas d'un réacteur, du disulfure organique ou du mercaptan avec du chlore et avec une solution aqueuse concentrée en HCl. Le sulfonyle formé est ensuite décanté du mélange réactionnel. Le principe du procédé étant ainsi connu depuis longtemps, les industriels cherchent à la rendre le plus économique possible, en jouant principalement sur le mode de circulation des liquides réagissants; tel est notamment l'objectif du brevet US n° 3 993 692 d'après lequel on fait arriver, en continu, du mercaptan $CH_3SH$, du chlore et une solution concentrée de HGl, par trois tubulures séparées, dans le fond d'un réacteur muni de chicanes transversales; entre ces dernières, tournent les pales d'un agitateur devant bien mélanger les réactifs introduits à l'état pulvérisé afin d'éviter la formation de grosses bulles; par ces moyens, on cherche à rendre la réaction complète et rapide. Dans le même but, le brevet français n° 1 598 279 préconise l'introduction séparée d'un disulfure ou d'un mercaptan et du chlore dans la solution chlorhydrique présente au fond du réacteur, de manière à produire un dégagement violent de HGl gazeux vers le haut, et engendrer ainsi une turbulence dans la zone de réaction.

Bien que ces méthodes de la technique connue permettent de réaliser la réaction rapidement, elles comportent des sujétions et difficultés technologiques, et – surtout en ce qui concerne le brevet français précité – ne permettent pas d'éviter toujours la formation de «cheminées» des courants de HCl, préjudiciables à la régularité du travail et aux bons rendements.

La présente invention apporte un perfectionnement qui rend possible l'obtention d'un milieu réactionnel parfaitement homogène, assurant un contact très intime entre les substances réagissantes. Grâce à ce perfectionnement, la fabrication se poursuit avec régularité, et peut être réalisée dans des appareils simples, sans chicanes ni turbulence, mais au moyen de machines tournantes dont le mixage obtenu conduit à d'excellents rendements.

Le procédé suivant l'invention est caractérisé en ce que la zone de réaction est alimentée en continu d'une part avec une émulsion stabilisée préalablement formée de disulfure organique dans de l'eau et/ou une solution chlorhydrique aqueuse de HCl et d'autre part avec du chlore. Le milieu réactionnel est continuellement agité mécaniquement et soutiré, pour être conduit rapidement à travers un système de refroidissement.

Le passage dans ce dernier système a lieu à une vitesse telle qu'il ne puisse pas y avoir séparation en deux couches et permet les transferts thermiques nécessaires.

De là, une part du liquide est envoyé dans une zone de décantation d'où le chlorure d'alcane sulfonyle est recueilli, tandis que la phase aqueuse chlorhydrique va vers la récupération de la solution chlorhydrique et de l'HCl gazeux.

Suivant un trait préféré de l'invention, au moins deux zones successives de décantation sont utilisées, la seconde recevant la phase aqueuse de la première. Le sulfonyle, entraîné par cette phase, est séparé dans la seconde zone et il est réuni à celui que l'on a recueilli à partir de la première zone de décantation.

Contrairement à ce qui est pratiqué dans l'art antérieur, la forme préférée de l'invention comprend la circulation du liquide réactionnel de haut en bas, à travers la zone de réaction, et non dans le sens ascendant. Cela est d'autant plus avantageux que le chlorure d'alcane sulfonyle est plus dense.

La condition principale de l'invention, c'est-à-dire la formation préalable d'une émulsion de disulfure organique avec de l'eau et/ou une solution aqueuse chlorhydrique, peut être réalisée par tous moyens connus, mais les gouttelettes de l'émulsion doivent être aussi fines que possible, en tout cas d'un diamètre inférieur à 100 microns, et, de préférence, ne dépassant pas 20 microns. Des émulsions à gouttelettes de 0,1 à 10 microns conviennent particulièrement bien. Ces émulsions peuvent être du type disulfure dans phase aqueuse, phase aqueuse dans disulfure ou les deux à la fois. Lorsqu'il s'agit de diméthyl-disulfure, dont la densité à 15° est de 1,057, donc égale à celle d'une solution aqueuse de 125 g HCl/l et voisine de celle de l'eau, et la tension superficielle assez faible par rapport à cette solution chlorhydrique et à l'eau, l'émulsification peut s'effectuer aisément par une agitation mécanique ou/et une circulation rapide en cycle fermé, dans un dispositif de type mélangeur statique par exemple.

Suivant ce dernier trait particulier de l'invention, il est possible d'émulsifier intimement le disulfure avec la solution chlorhydrique et/ou l'eau, quelles que soient les densités relatives de ces liquides.

Pour compléter l'émulsification on fait recirculer la majeure partie du milieu réactionnel, continuellement, entre la zone de réaction et la zone de décantation. A titre d'exemple, ce résultat peut être obtenu par la circulation, dans un circuit comprenant un échangeur, du liquide réactionnel, dont seulement ⅙ à ⅓ est envoyé, en continu, à la décantation. On produit ainsi une agitation puissante, qui empêche toute séparation en deux couches, avant que le liquide n'atteigne la zone de décantation.

Il peut cependant être utile de créer une émulsion très fine par l'adjonction d'un agent tensioactif. Ce dernier peut être choisi dans une quelconque des classes de tensioactifs connus, notamment cationiques ou non ioniques, par exemple sels de sulfonium ou d'ammonium quaternaire, ou bien de polyoxyalkylènes, actifs en milieu chlorhydrique. Il y a lieu toutefois de tenir compte de la nature chimique de cet agent en regard des matières réagissantes du procédé. Ainsi, des tensioactifs destructibles par el chlore sont recommandables, parce qu'ils permettent d'émulsionner le mélange disulfure-eau-HCl avant la chloration, et disparaissent peu à peu pendant cette réaction, ce qui évite les risques d'émulsification ultérieure du chlorure d'alcane sulfonyle produit, qu'il s'agit justement de séparer de la phase aqueuse. De tels agents se trouvent dans les groupes d'ammoniums quaternaires et de polyoxyalkylènes. Une autre solution consiste à employer un mercaptan tensioactif, tel que par exemple un polyéthoxy-mercaptan ou dodécyl-mercaptan qui peut conduire, au cours de la réaction à des produits intermédiaires ne gênant pas l'obtention du sulfonyle. Ce tensioactif, s'il reste dans le sulfonyle fabriqué, présente une masse moléculaire bien plus grande que celle du sulfonyle, et n'affecte pas la purification de celui-ci.

De toute manière, étant donné qu'il suffit d'une teneur très faible, de l'ordre de 0,001 à 0,1%, en agent tensioactif, l'addition d'une telle substance ne se répercute pas sur la pureté du chlorure d'alcane sulfonyle fabriqué.

Comme les procédés connus, celui de l'invention peut être pratiqué à des températures d'environ −10° à +60°C, et, de préférence, entre 0° et 20°C ou mieux entre 5° et 10°C. La réaction s'effectue à la pression atmosphérique, mais il peut être bon d'opérer sous une pression pouvant atteindre plusieurs bars, notamment lorsqu'on emploie un léger excès de chlore; les pressions préférées sont de l'ordre de 1,1 à 1,5 bars absolus. Bien que la proportion stoechiométrique de $Cl_2$ par rapport au disulfure convienne, c'est-à-dire 5 moles $Cl_2$ pour 1 mole de RSSR suivant la réaction

$$RSSR + 5Cl_2 + 4H_2O \xrightarrow{HCl} 2RSO_2Cl + 8HCl$$

on peut avoir intérêt à utiliser un faible excès de $Cl_2$, ne dépassant pas 5%, par rapport à cette proportion. Il est, par contre, recommandable de travailler avec un excès d'eau, c'est-à-dire avec 1 à 4 fois la quantité d'eau indiquée par l'équation ci-dessus, des quantités 1,1 à 3 fois celle-ci conviennent le mieux, ce qui représente 4,4 à 12 moles $H_2O$ par mole de disulfure.

Les concentrations préférées en HCl dans la phase aqueuse sont d'environ 10 à 45% et surtout 25 à 37%.

L'appareillage pour la réalisation du procédé suivant l'invention comprend, tout comme les installations connues, un ou plusieurs réacteurs dont la sortie est reliée à un système de refroidissement et, en aval de celui-ci, un dispositif de séparation par décantation; des moyens sont prévus pour le recyclage d'une fraction de l'acide chlorhydrique recueilli à la suite du séparateur. Conformément à l'invention, cette installation est caractérisée en ce qu'un générateur d'émulsion se trouve en amont du réacteur, la sortie de cet émulseur étant reliée à l'entrée du réacteur. L'alimentation de l'émulseur comprend une canalisation d'amenée de disulfure organique et une arrivée d'eau et/ou de solution aqueuse d'HCl provenant d'un courant de recyclage de récupération de l'HCl.

De préférence, le départ de l'émulsion est constitué par une tubulure branchée au fond du générateur, cette tubulure étant en communication avec le haut du réacteur.

A titre d'illustration, non limitative, la figure unique, annexée, représente schématiquement une installation pour la réalisation de l'invention.

Sur le dessin, 1 représente le corps d'un émulseur renfermant un agitateur 2 qui peut être par exemple un turbomélangeur. Les matières premières sont introduites dans l'émulseur, en continu, le disulfure par la canalisation 3, la solution chlorhydrique ou l'eau par 4; elles sont vigoureusement mélangées à l'aide de l'agitateur 2, de façon à donner une émulsion fine. Pour parfaire l'effet d'émulsion, on a prévu une pompe 5 qui reprend continuellement le liquide du fond de l'appareil et le renvoie, par le tuyau 6, en haut de l'émulseur.

Une pompe 7 envoie l'émulsion de 1, avec un débit continu bien déterminé, par la canalisation 8, dans la partie supérieure du réacteur 9, où arrive également le chlore par la tubulure 12. Le réacteur 9 est muni d'une double enveloppe de refroidissement 11 et d'un agitateur 10.

La réaction ayant eu lieu au moins en majeure partie dans le réacteur 9, le contenu de celui-ci est gardé à niveau constant et évacué en continu par le tube 14, au moyen de la pompe 15, et passe dans les tubes 17 d'un échangeur de chaleur 16 prévu pour une circulation rapide, empêchant la séparation du sulfonyle produit et favorisant les échanges therniques. La température étant ramenée à la valeur voulue et la réaction ayant pu se terminer dans cet échangeur 16-17, le liquide est conduit par 18 dans un premier décanteur 19, dont le fond communique par 21 avec un réceptacle 23 de sulfonyle produit.

Afin de maintenir le milieu réactionnel à l'état d'une émulsion homogène, qui n'ait pas tendance à se séparer en deux couches dans l'échangeur 16-17, une recirculation abondante est prévue par la dérivation 38 avant l'entrée du premier décanteur 19. Ainsi, une relativement faible partie du liquide, arrivant par 18, est introduite dans le décanteur 19; la majeure partie soit ⅚ à ⅔, est acheminée par 38 vers la ligne 14 ou l'entrée du réacteur 9 pour repasser, sous l'effet de la pompe 15, dans l'échangeur 16-17.

Du haut du décanteur 19, la phase liquide supérieure monte dans un second décanteur 20 où la séparation du sulfonyle est parachevée; du fond de 20, le produit est recueilli par 22 dans le réceptacle commun 23. Ce second décanteur 20 est placé sous une certaine dépression, dans le but de faire dégazer de l'HCl produit, contenu dans la solution aqueuse, et d'abaisser la concentration de celle-ci en acide; pratiquement, la dépression convenable est de l'ordre de 10 à 500 millibars et la concentration de la solution, après dégazage d'HCl, d'environ 25 à 35%.

Les gaz et vapeurs, dégazés ainsi du décanteur 20, sont acheminés par le tuyau 25, en haut d'un éjectolaveur 26 dont l'eau d'absorption est pompée par la tubulure 27 et joue le rôle de fluide moteur. L'acide HCl technique, ainsi obtenu dans 26, est recueilli dans une recette 29, tandis qu'une fraction peut en être dérivée par 30 vers un évaporateur 31 en vue de sa purification d'où de l'HCl purifié est reçu dans le récipient 32.

La phase aqueuse de concentration abaissée en HCl, sortant du décanteur 20, s'écoule par la canalisation 24, pour être recyclée en partie dans la boucle de recirculation 38-14-15-16-17-18, par 37, à l'aspiration de la pompe 15 et/ou à l'entrée du réacteur 9; le reste du liquide passe par 24' et 33 vers une installation de purge et de neutralisation 36.

Dans le cas où la récupération de l'acide méthane-sulfonique est envisagée, on pratique une dérivation sur la canalisation 24', pour soutirer de la solution qui est ensuite envoyée dans un dispositif d'hydrolyse du chlorure de méthane sulfonyle en vue de la fabrication du $CH_3SO_3H$.

Le repère 36 désigne globalement une installation de neutralisation de tous les effluents gazeux et liquides issus des différentes parties de l'installation.

L'exemple non limitatif, qui suit, montre le fonctionnement d'une installation pilote conçue selon la description donnée plus haut.

Exemple

Production, en continu, de chlorure de méthane sulfonyle.

Dans un émulseur 1 en acier inoxydable, de 60 litres de capacité, on fait arriver par 3 un débit constant de disulfure $CH_3SSCH_3$ de 2,5 kg/h, soit 26,5 moles/h, et par 4, une solution aqueuse à 25% HCl avec un débit représentant 5,7 kg/h d'eau, c'est-à-dire 317 moles $H_2O$/heure. La proportion de l'eau, par rapport au disulfure, est donc 3 fois celle de la stoechiométrie. On opère à la température ambiante. La pompe 5 débite 1000 l/h, ce qui produit un fort brassage du mélange liquide, le contenu de l'émulseur étant recirculé 17 fois (1000:60 = 16,66). On obtient ainsi une émulsion fine et stable, dont 80% de gouttelettes ont des diamètres de 1 à 20 microns. Abstraction faite de l'HCl, la composition de cette émulsion est en poids de 31,5% $CH_3SSCH_3$ et 68,5% $H_2O$. Le réacteur 9, en acier chemisé de verre intérieurement, a une capacité de 100 l. La pompe 7 envoie, dans le réacteur 9, 8,25 kg d'émulsion par heure; en même temps, on fait arriver, directement dans le liquide, un débit horaire de 9,4 à 9,7 kg de chlore gazeux par la conduite 12 munie d'un diffuseur; le premier de ces nombres correspond au rapport stoechiométrique, le second à un excès de 3%.

L'agitateur 10 à trois pales, tourne à 150 tours/mn. La température du mélange réactionnel, dans le réacteur 9, est maintenue entre 7° et 10°C, par circulation d'un fluide qui entre à −8°C dans la double enveloppe 11.

Une pression de 1,5 bar absolu est maintenue dans le réacteur 9. L'échangeur tubulaire 16-17, en acier doublé de verre, a une capacité utile de 60 l; il reçoit par la pompe 15, à partir du réacteur 9, un débit de 6 à 8 $m^3$/h de mélange réactionnel, qu'il maintient à une température de 5 à 10°C. Une fraction de 1 à 2 $m^3$, soit ⅙ à ⅓ de ce débit passe dans le décanteur 19, le reste constituant la masse de recirculation d'agitation par 38-14-15-16-17-18, cette masse arrivant par partie en pied et par partie en tête du réacteur 9.

Le mélange subit ensuite la séparation dans les deux décanteurs en série 19 et 20 qui laissent couler dans le réceptacle 23 le sulfonyle produit, à raison de 5,65 à 5,80 kg/h, ce qui correspond à un rendement de 92,5 à 95%.

L'acide chlorhydrique, récupéré de la phase gazeuse du décanteur 20, purifié en 31 et recueilli en 39, est recyclé en partie vers l'émulseur 1 par la canalisation 35. Il fournit ainsi la solution aqueuse, chlorhydrique, pour la préparation de l'émulsion décrite plus haut.

2 à 8 kg par heure de liquide aqueux du décanteur 20 sont purgés par la canalisation 24-24'-33, le reste étant recyclé vers la boucle de réaction.

La transformation du disulfure de départ est pratiquement complète.

Le produit brut, obtenu, a la composition moyenne:

| | |
|---|---|
| chlorure de méthane sulfonyle | 98 à 98,5% |
| HCl | 1 à 1,5 |
| acide méthane sulfonique | environ 0,5 |
| diméthyl-disulfure | traces |

Après distillation, on recueille du chlorure de méthane sulfonyle à 99,8%, ne contenant pas plus de 0,18% d'HCl et 100 ppm de disulfure.

L'invention comprend également la récupération de l'acide méthane sulfonique, présent en tant que sous-produit, dans la phase aqueuse chlorhydrique du procédé.

## Revendications

1. Procédé de fabrication continue d'un chlorure d'alcane sulfonyle par l'action du chlore sur le disulfure de dialkyle correspondant à cet alcane, en présence d'eau ou d'une solution aqueuse d'acide chlorhydrique, dans lequel on prépare d'abord, en continu, une émulsion de disulfure de dialkyle avec de l'eau et/ou la solution chlorhydrique par une agitation mécanique, et que l'on introduit ensuite, également en continu, le chlore dans l'émulsion formée, caractérisé en ce que les gouttelettes de l'émulsion ont des diamètres inférieurs à 100 microns, et en ce que ladite agitation est complétée par la recirculation continuelle du milieu réactionnel entre une zone de réaction de chloration du disulfure de dialkyle et une zone de décantation.

2. Procédé suivant la revendication 1, caractérisé en ce que les gouttelettes de l'émulsion ont des diamètres ne dépassant pas 20 microns.

3. Procédé suivant la revendication 1 ou 2, caractérisé en ce que le milieu est additionné de 0,001 à 0,1% d'un agent tensio-actif.

4. Procédé suivant une des revendications 1 à 3, caractérisé en ce que la préparation de l'émulsion et la réaction avec le chlore sont effectuées en deux zones distinctes, l'émulsion étant transférée en continu de sa zone de préparation (1) dans la zone de chloration (9).

5. Procédé suivant une des revendications 1 à 4, dans lequel le mélange réactionnel, sortant en continu de la zone de réaction (9) où il est agité, passe dans un système de refroidissement (16–17), caractérisé en ce que ce passage a lieu avec une vitesse telle qu'il n'y ait pas de séparation de phases au cours du refroidissement.

6. Procédé suivant une des revendications 1 à 5, caractérisé en ce que la majeure partie du milieu réactionnel entre la zone de réaction et la zone de décantation est soumise à une recirculation continuelle.

7. Procédé suivant la revendication 5, dans lequel le mélange réactionnel, sortant du système de refroidissement, est soumis à la décantation, caractérisé en ce que celle-ci a lieu dans au moins deux enceintes (19, 20) distinctes, dont la seconde (20) est maintenue sous une pression réduite.

8. Procédé suivant la revendication 7, comprenant le recyclage d'une partie de la solution aqueuse chlorhydrique vers la zone de refroidissement (16–17), caractérisé en ce que cette solution est prélevée dans la phase liquide supérieure, décantée dans la seconde enceinte (20) et que son recyclage (24–37) est dirigé vers l'aspiration d'une pompe (15) avec le réacteur (9), ces courants étant véhiculés vers la zone de refroidissement (16–17).

9. Procédé suivant une des revendications précédentes, dans lequel l'acide chlorhydrique décanté est réutilisé, caractérisé en ce que cet acide provient du dégazage de la phase aqueuse dans le décanteur (20) travaillant sous dépression, et subit une purification (26–30–31) avant d'être envoyé (39–35) dans la zone d'émulsion (1).

10. Procédé suivant une des revendications 1 à 9, caractérisé en ce que le chlorure d'alcane sulfonyle préparé est le chlorure de méthane sulfonyle.

11. Procédé suivant une des revendications précédentes, caractérisé en ce que l'acide alcane sulfonique, présent dans la solution chlorhydrique après la réaction, est récupéré par hydrolyse à partir de la purge 24'.

12. Procédé suivant une des revendications 1 et 4 à 6, caractérisé en ce que le milieu réactionnel circule de haut en bas à travers la zone de réaction.

13. Appareillage pour la réalisation du procédé suivant une des revendications précédentes, qui comprend un réacteur (9), un échangeur de chaleur (16–17) en aval du réacteur, et des moyens de décantation (19) reliés à la sortie de l'échangeur, caractérisé en ce que le réacteur (9) est précédé par émulseur (1) dont la sortie (8) communique avec l'entrée du réacteur.

14. Appareillage suivant la revendication 13, caractérisé en ce que les moyens de décantation comprennent au moins deux décanteurs (19, 20), montés en série, dont le second (20) est muni de moyens pour fonctionnement sous dépression.

15. Appareil suivant la revendication 13 ou 14, caractérisé en ce que le circuit entre le réacteur (9), le décanteur (19) et l'échangeur (16–17), comprend une boucle de recirculation (38–14–15–16–17–18) permettant de faire circuler très rapidement la majeure partie du milieu réactionnel, dont une fraction seulement est dirigée en continu dans le décanteur (19).

## Patentansprüche

1. Verfahren zur kontinuierlichen Herstellung eines Alkansulfonylchlorids durch Einwirken von Chlor auf das diesem Alkan entsprechende Dialkyldisulfid, in Anwesenheit von Wasser oder einer wässrigen Lösung von Chlorwasserstoffsäure, bei dem man zuerst kontinuierlich eine Dialkyldisulfidemulsion mit dem Wasser und/oder der Chlorwasserstofflösung durch mechanisches Rühren herstellt und anschliessend ebenfalls kontinuierlich das Chlor in die gebildete Emulsion einführt, dadurch gekennzeichnet, dass die Tröpfchen der Emulsion Durchmesser von unter 100 Mikron aufweisen und dass das mechanische Rühren durch kontinuierliches Rezirkulieren des Reaktionsmediums zwischen einer Chlorierungsreaktionszone des Dialkyldisulfids und einer Dekantierzone vollendet wird.

2. Verfahren nach Anspruch 1, dadurch gekennzeichnet, dass die Tröpfchen der Emulsion Durchmesser aufweisen, die 20 Mikron nicht überschreiten.

3. Verfahren nach Anspruch 1 oder 2, dadurch gekennzeichnet, dass 0,001 bis 0,1% eines oberflächenaktiven Wirkstoffs dem Medium beigefügt wird.

4. Verfahren nach einem der Ansprüche 1 bis

3, dadurch gekennzeichnet, das die Herstellung der Emulsion und die Reaktion mit dem Chlor in zwei unterschiedlichen Zonen durchgeführt werden, wobei die Emulsion kontinuierlich von ihrer Herstellungszone (1) in die Chlorierungszone (9) übergeführt wird.

5. Verfahren nach einem der Ansprüche 1 bis 4, bei dem das Reaktionsgemisch, das kontinuierlich aus der Reaktionszone (9), wo es gerührt wird, kommt, in ein Abkühlsystem (16-17) fliesst, dadurch gekennzeichnet, dass dieses Fliessen mit einer derartigen Geschwindigkeit erfolgt, dass keine Phasentrennung im Verlauf des Abkühlens auftritt.

6. Verfahren nach einem der Ansprüche 1 bis 5, dadurch gekennzeichnet, dass der Hauptanteil des Reaktionsmediums zwischen der Reaktionszone und der Dekantierzone einer kontinuierlichen Rezirkulation unterworfen wird.

7. Verfahren nach Anspruch 5, bei dem das Reaktionsgemisch, das aus dem Abkühlsystem kommt, einem Dekantieren unterzogen wird, dadurch gekennzeichnet, dass dies in mindestens zwei unterschiedlichen Orten (19, 20) erfolgt, von denen der zweite (20) unter einem verringerten Druck gehalten wird.

8. Verfahren nach Anspruch 7 unter Recyclisieren eines Teils der wässrigen Chlorwasserstofflösung zur Abkühlzone (16-17) hin, dadurch gekennzeichnet, dass diese Lösung in der oberen flüssigen Phase entnommen wird, die in dem zweiten Ort (2) dekantiert wird und dass ihre Recyclisierung (24-37) zur Ansaugverbindung einer Pumpe (15) mit dem Reaktor (9) hin gerichtet wird, wobei diese Ströme zur Abkühlzone (16-17) geführt werden.

9. Verfahren nach einem der vorhergehenden Ansprüche, bei dem die dekantierte Chlorwasserstoffsäure erneut verwendet wird, dadurch gekennzeichnet, dass diese Säure von dem Entgasen der Wasserphase in der Dekantiervorrichtung (20) kommt, die unter verringertem Druck arbeitet, und einer Reinigung (26-30-31) unterzogen wird, bevor sie zur Emulsionszone (1) geschickt (39-35) wird.

10. Verfahren nach einem der Ansprüche 1 bis 9, dadurch gekennzeichnet, dass das hergestellte Alkansulfonylchlorid Methansulfonylchlorid ist.

11. Verfahren nach einem der vorhergehenden Ansprüche, dadurch gekennzeichnet, dass die in der Chlorwasserstofflösung nach der Reaktion vorhandene Alkansulfonsäure durch Hydrolyse, ausgehend von der Reinigung (24′) wiedergewonnen wird.

12. Verfahren nach einem der Ansprüche 1 und 4 bis 6, dadurch gekennzeichnet, dass das Reaktionsmedium von oben nach unten durch die Reaktionszone zirkuliert.

13. Vorrichtung zur Durchführung des Verfahrens gemäss einem der vorhergehenden Ansprüche, enthaltend einen Reaktor (9), einen Wärmeaustauscher (16-17) stromabwärts vom Reaktor, und Einrichtungen zum Dekantieren (19), die mit dem Ausgang des Austauschers verbunden sind, dadurch gekennzeichnet, dass dem Reaktor (9) ein Emulgator (1) vorausgeht, dessen Ausgang (8) mit dem Eingang des Reaktors in Verbindung steht.

14. Vorrichtung nach Anspruch 13, dadurch gekennzeichnet, dass die Einrichtungen zum Dekantieren mindestens zwei Dekantiervorrichtungen (19, 20), die in Serie montiert sind, umfasst, von denen die zweite (20) mit Einrichtungen zum Betrieb unter verringertem Druck ausgerüstet ist.

15. Vorrichtung nach Anspruch 13 oder 14, dadurch gekennzeichnet, dass die Leitung zwischen dem Reaktor (9), der Dekantiervorrichtung (19) und dem Austauscher (16-17) eine Rezirkulationsschleife (38-14-15-16-17-18) umfasst, die eine sehr rasche Zirkulation des Hauptteils des Reaktionsmediums ermöglicht, von dem nur ein Teil kontinuierlich in die Dekantiervorrichtung (19) gerichtet wird.

## Claims

1. Continuous process of manufacture of an alkane sulphonyl chloride by the action of chlorine on the dialkyl disulphide corresponding to the alkane, in the presence of water or an aqueous hydrochloric acid solution, in which an emulsion of the dialkyl disulphide with water and/or the hydrochloric acid solution is first prepared continuously by mechanical agitation and chlorine is then introduced, likewise continuously, into the emulsion formed, characterized in that the droplets of the emulsion have diameters below 100 microns and that the agitation is completed by continuous recirculation of the reaction medium between a reaction zone of chlorination of the dialkyl disulphide and a decantation zone.

2. Process according to claim 1, characterized in that the droplets of the emulsion have diameters not exceeding 20 microns.

3. Process according to claim 1 or 2, characterized in that 0.001 to 0.1% of a surface active agent is added to the medium.

4. Process according to any of claims 1 to 3, characterized in that the preparation of the emulsion and the reaction with chlorine are effected in two separate zones, the emulsion being continuously transferred from its preparation zone (1) to the chlorination zone (9).

5. Process according to any of claims 1 to 4, in which the reaction mixture, continuously leaving the reaction zone (9) where it is agitated, passes into a cooling system (16-17), characterized in that this transfer takes place at a speed such that there is no separation of the phases during the cooling.

6. Process according to any of claims 1 to 5, characterized in that the major part of the reaction medium between the reaction zone and the decantation zone is subjected to continuous recirculation.

7. Process according to claim 5, in which the reaction medium leaving the cooling system is subjected to decantation, characterized in that this takes place in at least two separate enclosures (19, 20), the second (20) of which is maintained under a reduced pressure.

8. Process according to claim 7, comprising the recycling of a part of the aqueous hydrochloric acid solution to the cooling zone (16–17), characterized in that this solution is introduced into the upper liquid phase, decanted in the second enclosure (20) and its recycling (24–37) is directed towards the inlet of a pump (15) with the reactor (9), these streams being directed toward the cooling zone (16–17).

9. Process according to any of the preceding claims, in which the hydrochloric acid decanted is re-used, characterized in that this acid causes degassing of the aqueous phase in the decanter (20) operating under subpressure and undergoes purification (26–30–31) before being transmitted (39–35) into the emulsion zone (1).

10. Process according to any of claims 1 to 9, characterized in that the alkane sulphonyl chloride prepared is methane sulphonyl chloride.

11. Process according to any of the preceding claims, characterized in that the alkane sulphonic acid present in the hydrochloric acid solution after the reaction is recovered by hydrolysis starting at the purge 24′.

12. Process according to any of claims 1 and 4 to 6, characterized in that the reaction medium circulates from top to bottom through the reaction zone.

13. Apparatus for carrying out the process according to any of the preceding claims, which comprises a reactor (9), a heat exchanger (16–17) downstream of the reactor and decantation means (19) connected to the outlet from the exchanger, characterized in that the reactor (9) is preceded by an emulsifier (1), the outlet (8) of which communicates with the inlet to the reactor.

14. Apparatus according to claim 13, characterized in that the decantation means comprise at least two decanters (19, 20) mounted in series, the second (20) of which is provided with means for operating under sub-pressure.

15. Apparatus according to claim 13 or 14, characterized in that the circuit between the reactor (9), the decanter (19) and the exchanger (16–17), comprises a recirculation loop (38–14–15–16–17–18) allowing very rapid circulation of the major part of the reaction medium, only a fraction of which is continuously directed into the decanter (19).

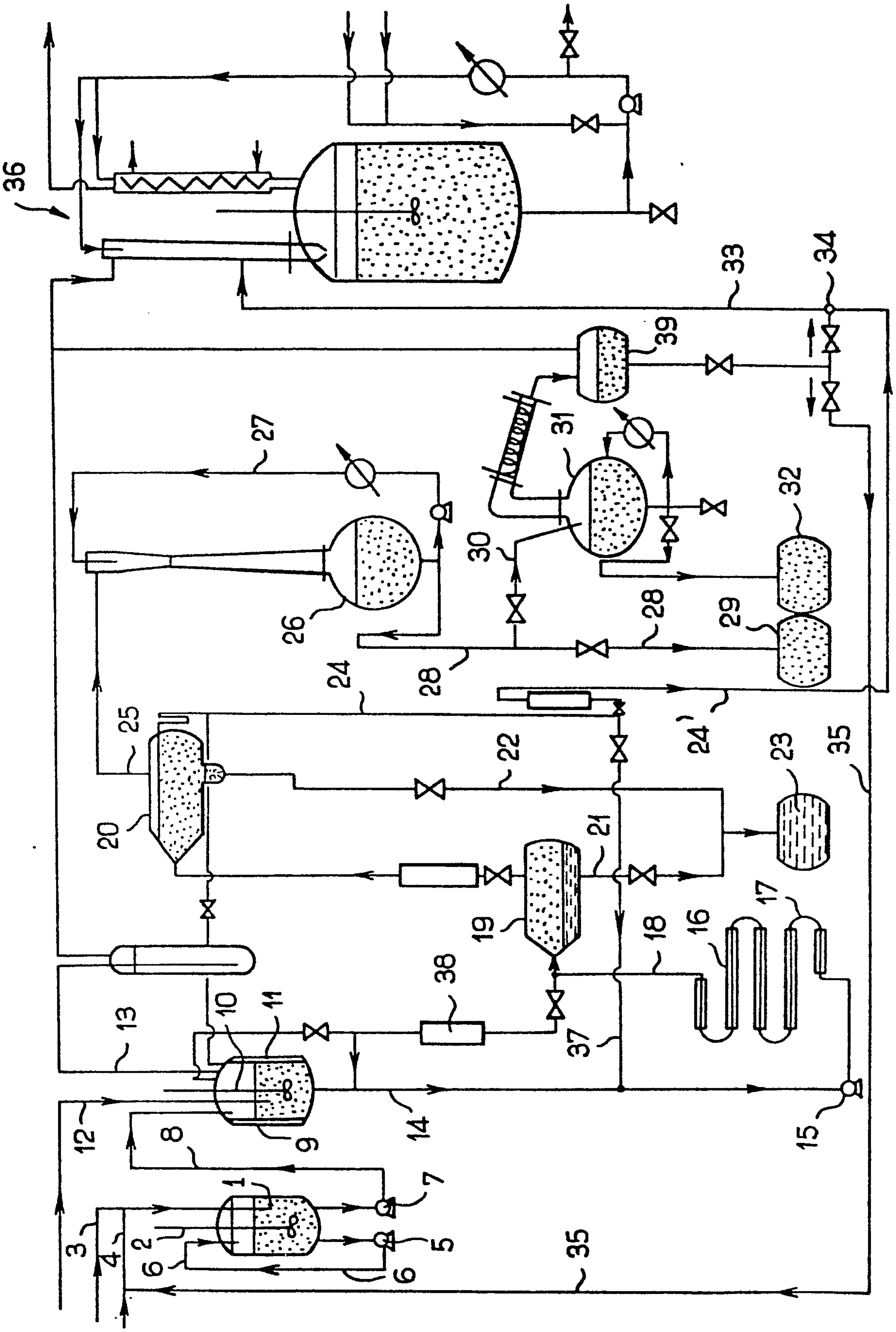
1
2
3
4
5
6
7
8
9
10
11
12
13
14
15
16
17
18
19
20
21
22
23
24
24'
25
26
27
28
29
30
31
32
33
34
35
36
37
38
39